# EUROPÄISCHE PATENTANMELDUNG

(11) **EP 1 695 975 A1**
(43) Veröffentlichungstag der Anmeldung: **30.08.2006**
(21) Anmeldenummer: 06010738.0
(22) Anmeldetag: 08.04.2000
(51) Int. Cl.: C07D 475/04, A61K 31/519, A61P 39/02

(54) **Stabile kristalline Salze von 5-Methyltetrahydrofolsäure**

(30) Priorität: 15.04.1999 CH 69599
(62) Teilanmeldung aus: 00107623.1
(71) Anmelder: Merck Eprova AG, 8200 Schaffhausen (CH)
(72) Erfinder: Müller, Hans Rudolf, 8207 Schaffhausen (CH); Egger, Thomas, 8307 Effretikon (CH); Moser, Rudolf, 8200 Schaffhausen (CH)

(57) **Zusammenfassung**

Die Erfindung betrifft stabile kristalline Salze der 5-Methyl-(6R,S)-, -(6S)- und -(6R)-tetrahydrofolsäure, Verfahren zur Herstellung dieser Salze sowie deren Verwendung als Bestandteil zur Herstellung von Arzneimitteln, als Nahrungsmittelergänzungsstoff und Zubereitungen enthaltend diese Salze.

## Beschreibung

Die Erfindung betrifft kristalline N-[4-[[(2-amino-1,4,5,6,7,8-hexahydro-4-oxo-5-methyl-(6S)-, -(6R)- und -(6R,S)-pteridinyl)methyl]amino]benzoyl]-L-glutaminsäure-Salze (im folgenden 5-Methyltetrahydrofolsäure-Salze genannt), deren Verwendung sowie ein Verfahren zu deren Herstellung.

Verwendet werden Tetrahydrofolate vorwiegend als 5-Formyltetrahydrofolsäure und deren Salze (Leucovorin) oder 5-Methyltetrahydrofolsäure und deren Salze zur Behandlung von megaloblastischer Folsäure-Anämie, als Antidot zur Verstärkung der Verträglichkeit von Folsäure-Antagonisten, speziell von Aminopterin und Methotrexat in der Krebstherapie ("Antifolate rescue"), zur Verstärkung des therapeutischen Effektes von fluorierten Pyrimidinen und der Behandlung von Autoimmunkrankheiten wie Psoriasis und rheumatischer Arthritis, zur Verstärkung der Verträglichkeit von bestimmten Antiparasitika, etwa Trimethoprim-Sulfamethoxazol sowie zur Verminderung der Toxizität von Dideazatetrahydrofolaten in der Chemotherapie. 5-Methyltetrahydrofolsäute wird speziell eingesetzt als Arzneimittel und als Nahrungsmittelergänzungsstoff, Vitaminpräparat, zur Prävention von Neuralrohrdefekten, zur Behandlung von depressiven Erkrankungen und zur Beeinflussung des Homocysteinspiegels.

5-Methyltetrahydrofolsäure und deren Salze sind extrem instabil, wobei insbesondere die hohe Oxidationsempfindlichkeit auffällt [siehe dazu auch A.L. Fitzhugh, Pteridines 4(4), 187-191 (1993)] und daher schwierig in einer für einen pharmazeutischen Wirkstoff oder Nahrungsmittelergänzungsstoff akzeptablen Reinheit herzustellen.

Zur Überwindung der Instabilität der 5-Methyltetrahydrofolsäure wurden verschiedene Methoden wie ein möglichst vollständiger Sauerstoffausschluss oder die Zugabe von Oxidationsschutzmitteln wie Ascorbinsäure oder reduziertes L-Glutathion angewendet. Ein vollständiger Sauerstoffausschluss ist jedoch bei der Verwendung kaum, und wenn dann nur mit sehr grossem Aufwand zu realisieren, und die Zugabe von Oxidationsschutzmitteln ist ebenfalls nicht immer möglich. Bisher konnte demnach noch kein technisch gangbares Verfahren gefunden werden, das für die Herstellung von ausreichend stabilen 5-Methyltetrahydrofolsäure-Salzen mit einer hohen Reinheit geeignet ist.

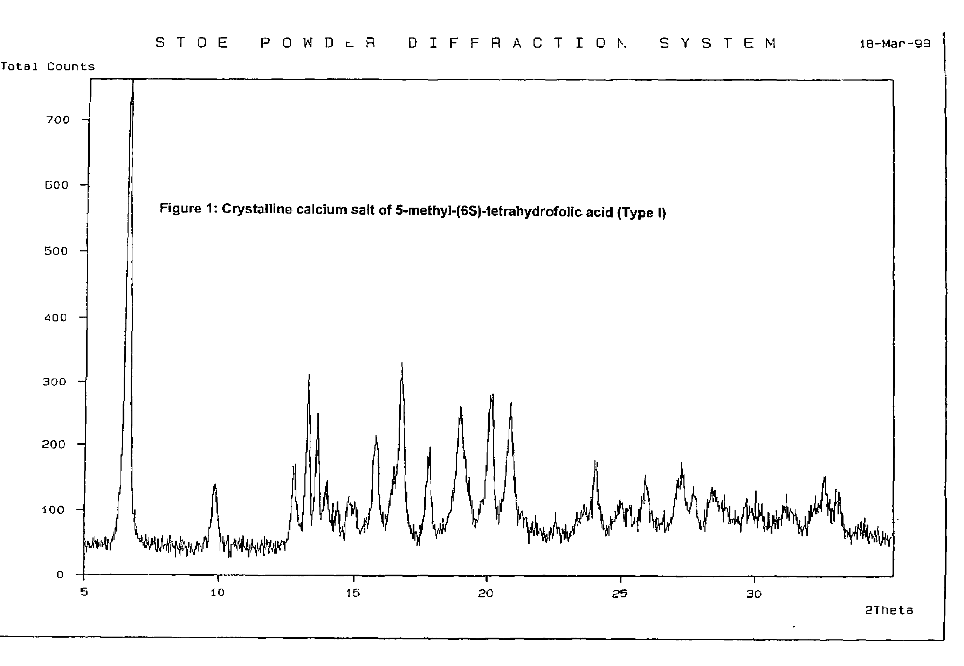

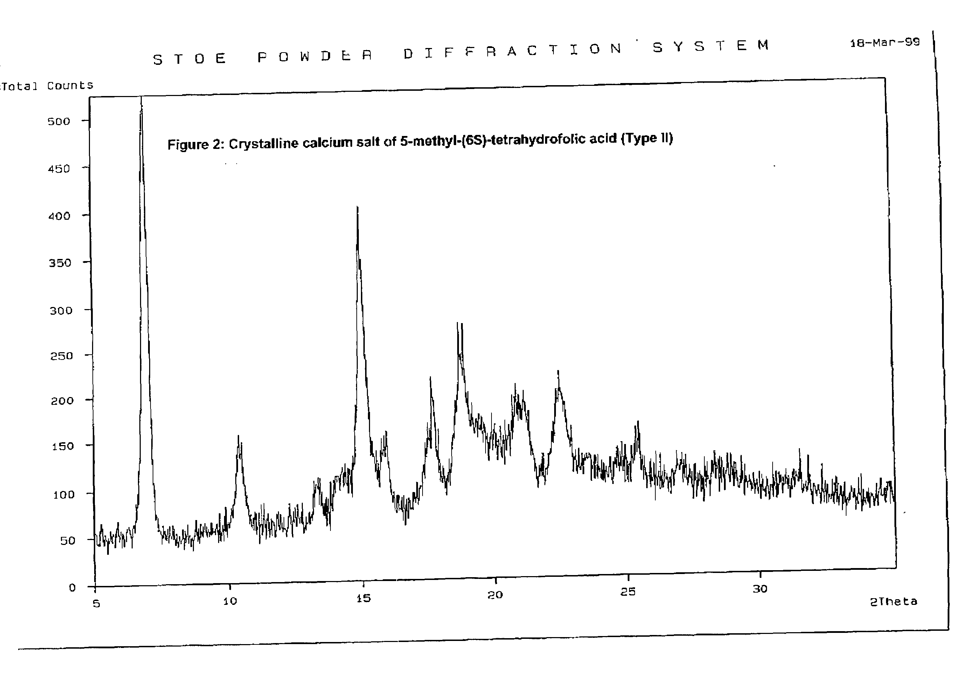

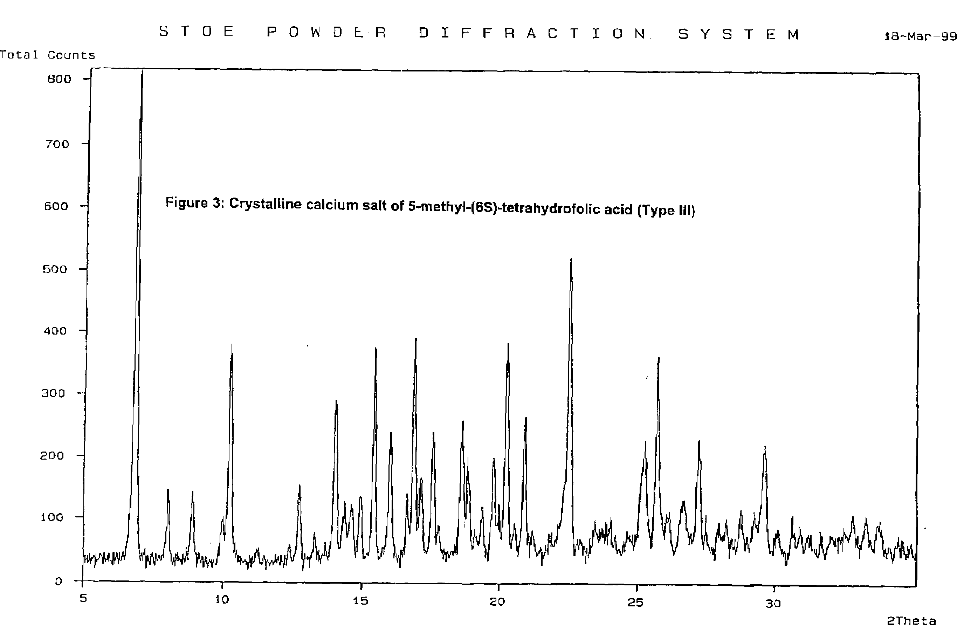

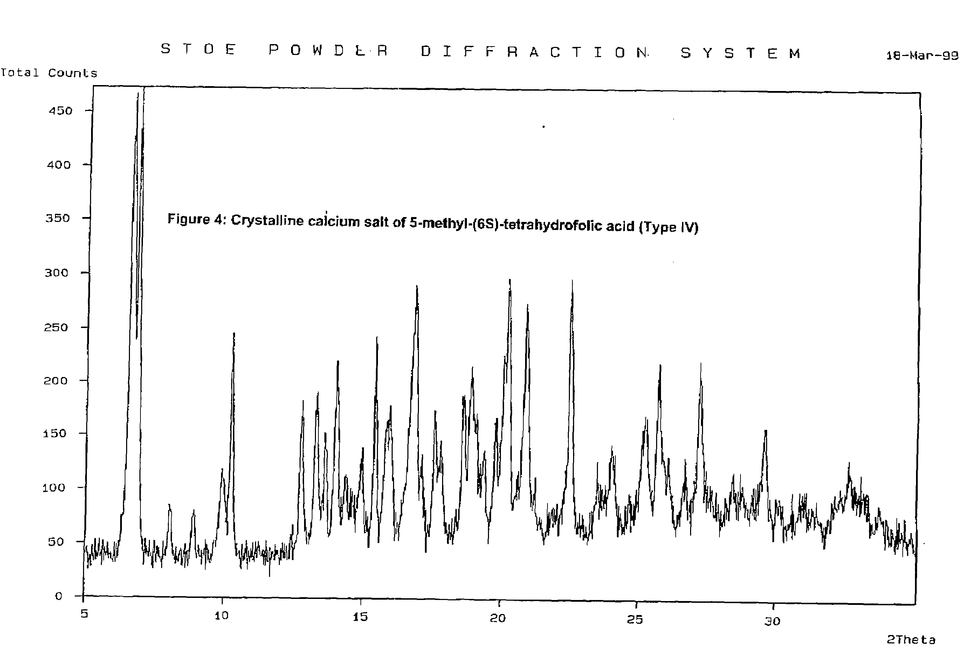

Es wurde nun überraschend gefunden, dass sich 5-Methyltetrahydrofolsäure-Salze mit einer hohen chemischen Reinheit und einer ausgezeichneten Stabilität erhalten lassen, indem das entsprechende Salz aus einem polaren Medium nach einer Temperaturbehandlung der Lösung von über 60°C kristallisiert wird. Die so erhaltenen hohen kristallinen 5-Methyltetrahydrofolsäure-Salze sind bei Raumtemperatur praktisch unbeschränkt stabil. Sie sind geeignet als Bestandteil oder als Ausgangsmaterial zur Herstellung von Arzneimittelformen oder Nahrungsmittelergänzungsstoffen.
Gegenstand der Erfindung sind demnach kristalline 5-Methyltetrahydrofolsäure-Salze. Als 5-Methyltetrahydrofolsäure-Salze zur Kristallisation bevorzugt eingesetzt werden Erdalkalisalze, im Speziellen das Calcium-Salz. Die kristallinen 5-Methyltetrahydrofolsäure-Salze weisen eine bisher nie erreichte Reinheit von > 98% zusammen mit einer bisher nie erreichten Stabilität von > 98% in Bezug auf den Ausgangswert nach 6 Monaten Lagerung unter Luft bei 25°C und 60% relativer Luftfeuchtigkeit auf. Die kristallinen 5-Methyl-(6S)-tetrahydrofolsäure-Calciumsalze liegen in vier verschiedenen Kristallmodifikationen vor (Typ I, Typ II, Typ III und Typ IV) und zeigen bei Pulverröntgendiffraktions-Messungen scharfe Banden (siehe dazu Figure 1 bis Figure 4 und Table 1 bis Table 4). Ausgewählte 2 Theta-Werte für die verschiedenen Kristallmoditikationen liegen bei 6.5, 13.3, 16.8 und 20.1 (Typ I); bzw. 5.3, 6.9, 18.7 und 21.1 (Typ II); bzw. 6.8, 10.2, 15.4 und 22.5 (Typ III), bzw. 6.6, 15.9, 20.2 und 22.5 (Typ IV). Kristalline 5-Methyltetrahydrofolsäure-Calciumsalze weisen einen Kristallwassergehalt von mindestens 1 Aequivalent Wasser auf 1 Aequivalent 5-Methyltetrahydrofolsäure auf. So enthält die Modifikation Typ I typischerweise ≥ 3 Aequivalente Wasser, die Modifikation Typ II typischerweise ≤ 2 Aequivalente Wasser und die Modifikationen Typ III und Typ IV typischerweise ≤ 3 Aequivalente Wasser.

5-Methyl-(6R)-tetrahydrofolsäure-Salze und 5-Methyl-(6R,S)-tetrahydrofolsäure-Salze können ebenfalls in hoher kristalliner Form erhalten werden.

Ein weiterer Gegenstand der Erfindung ist das Verfahren zur Herstellung hoher kristalliner Salze der 5-Methyltetrahydrofolsäure-Salze, welches dadurch gekennzeichnet ist, dass man das entsprechende Salz der 5-Methyltetrahydrofolsäure kristallisiert. Die Kristallisation der 5-Methyltetrahydrofolsäure-Salze erfolgt dabei aus einem polaren Medium nach einer Temperaturbehandlung von über 60°C, speziell über 85°C.

Als polares Medium eignen sich vor allem Wasser oder ein Gemisch aus Wasser und einem mit Wasser mischbaren organischen Lösungsmittel wie wasserlöslichen Alkoholen, z B. Methanol. Ethanol, n-Propanol, iso-Propanol, Ethylenglycol, einer niedrigen aliphatischen wasserlöslichen Carbonsäure, z. B. Ameisensäure, Essigsäure, Milchsäure oder wasserlöslichen Amiden, z. B. Formamid, Dimethylformamid, Dimethylacetamid, 1-Methylpyrrolidon, 2-Methylpyrrolidon, 2-Piperidinon. Es gibt keine besonderen Einschränkungen bezüglich der Art des eingesetzten Lösungsmittels und des Mischungsverhältnisses, da kristalline 5-Methyltetrahydrofolsäure-Salze generell tiefere Löslichkeiten als die entsprechenden amorphen Formen aufweisen.

Die Kristallisation wird bevorzugt aus Lösungen durchgeführt. Die Kristallisation aus einer Suspension ist aber ebenfalls möglich.

Durch weitere Temperaturbehandlungen von über 60°C unter kontrollierter Luftfeuchtigkeit lassen sich die verschieden Kristallmodifikationen ineinander umwandeln. So kann Typ I, hergestellt durch eine Kristallisation aus einem polaren Medium nach einer Temperaturbehandlung von über 60°C, durch Trocknen im Vakuum bei 70°C in Typ II, durch eine Temperaturbehandlung von über 90°C in Typ III und durch eine Temperaturbehandlung von über 95°C in Typ IV umgewandelt werden. Typ II lässt sich durch Behandlung mit Wasser in einer Feuchtekammer bei 90°C wieder in Typ I zurückführen.

Die Kristallisation der 5-Methyltetrahydrofolsäure-Salze erfolgt spontan oder durch Animpfen mit dem entsprechendem kristallinem 5-Methyltetrahydrofolsäure-Salz.

Als Ausgangsmaterial der Kristallisation eignet sich bevorzugt amorphe oder kristalline, reine 5-Methyl-(6S)- oder -(6R)-tetrahydrofolsäure, es kann jedoch auch racemische 5-Methyl-(6R,S)-tetrahydrofolsäure wie auch angereicherte 5-Methyl-(6S)-, -(6R)- oder -(6R,S)-tetrahydrofolsäure eingesetzt werden.

Durch den Einsatz von amorpher oder teilkristalliner optisch reiner 5-Methyltetrahydrofolsäure oder deren Salzen als Ausgangsmaterial für die Kristallisation erhält man durch das beschriebene Verfahren im Wesentlichen kristalline 5-Methyltetrahydrofolsäure-Salze von bisher nie erreichter Reinheit zusammen mit einer bisher nie erreichten Stabilität.

Die Erfindung betrifft auch die Verwendung hoher kristalliner 5-Methyltetrahydrofolsäure-Salze als Bestandteil zur Herstellung von Arzneimitteln oder Nahrungsmittelergänzungsstoffen oder zur Herstellung anderer Tetrahydrofolsäure-Derivate, da kristalline 5-Methyltetrahydrofolsäure-Salze aufgrund ihrer ausgezeichneten Stabilität in fester Form eine zeitlich praktisch unbeschränkt gleichbleibende sehr gute Qualität behält. Ebenso betrifft die Erfindung auch Zubereitungen enthaltend hohe kristalline 5-Methyltetrahydrofolsäure-Salze. Die Herstellung der Zubereitungen erfolgt nach bekannten Verfahren. Die Anwendung erfolgt analog zur Anwendung von bekannten Substanzen aus dem Gebiet der Tetrahydrofolate wie z. B. 5-Formyltetrahydrofolsäure (Leucovorin).

Die vorliegende Beschreibung ermöglicht es dem Fachmann die Erfindung umfassend anzuwenden. Die folgenden Beispiele sind deshalb nur zur Veranschaulichung möglicher Varianten und daher in keiner Art und Weise einschränkend zu verstehen.

Alle in den folgenden Beispielen erwähnten Temperaturen sind in Grad Celsius angegeben. Wo nicht anders bezeichnet sind Gehaltsangaben als Gewichts-% aufgeführt.

### Beispiele zur Illustrierung der Erfindung

Der in den Beispielen angegebene Gehalt an 5-Methyltetrahydrofolsäure-Salz wurde jeweils mit HPLC bestimmt und in %-Fläche angegeben, der Wassergehalt wurde über eine Karl-Fischer-Methode bestimmt.

### Beispiel 1 [Stabllitäten]

Zur Stabilitätsbestimmung der kristallinen 5-Methyltetrahydrofolsäure-Salze wurden die Substanzen zusammen mit Vergleichsmustern bei 25°C und 60% relative Feuchte unter Luft gelagert. In periodischen Abständen wurde der verbleibende Gehalt an 5-Methyltetrahydrofolsäure-Salz gemessen und im Vergleich zum Ausgangswert angegeben.

| | **Belastungszeit in Monaten** | | | | | |
|---|---|---|---|---|---|---|
| | **0** | **3** | **6** | **12** | **18** | **88** |
| kristallines Calcium-Salz der 5-Methyl-(6S)-tetrahydrofolsäure | 100% | 98.6% | 98.7% | 99.1% | 99.0% | 97.8% |
| amorphes Calcium Salz der 5-Methyl-(6S)-tetrahydrofolsäure | 100% | | | 84.2% | | |

Kristalline 5-Methyltetrahydrofolsäure-Salze sind auch nach längerer Belastungszeit noch sehr hell. Im Gegensatz dazu verfärben sich die amorphen Proben rasch sehr stark.

### Beispiel 2 [Pulverröntgendiagramme]

Zur Charakterisierung der strukturellen Eigenschaften (Kristallmodifikationen) der kristallinen 5-Methyltetrahydrofolsäure-Salze wurden von diesen Substanzen Pulverröntgendiagramme (Beugungsspektren) aufgenommen.

Kristalline 5-Methyltetrahydrofolsäure-Salze ergeben gut aufgelöste Spektren mit scharfen Banden und niedrigem Untergrund, Die Spektren weisen auf hohe kristalline Anteile hin.

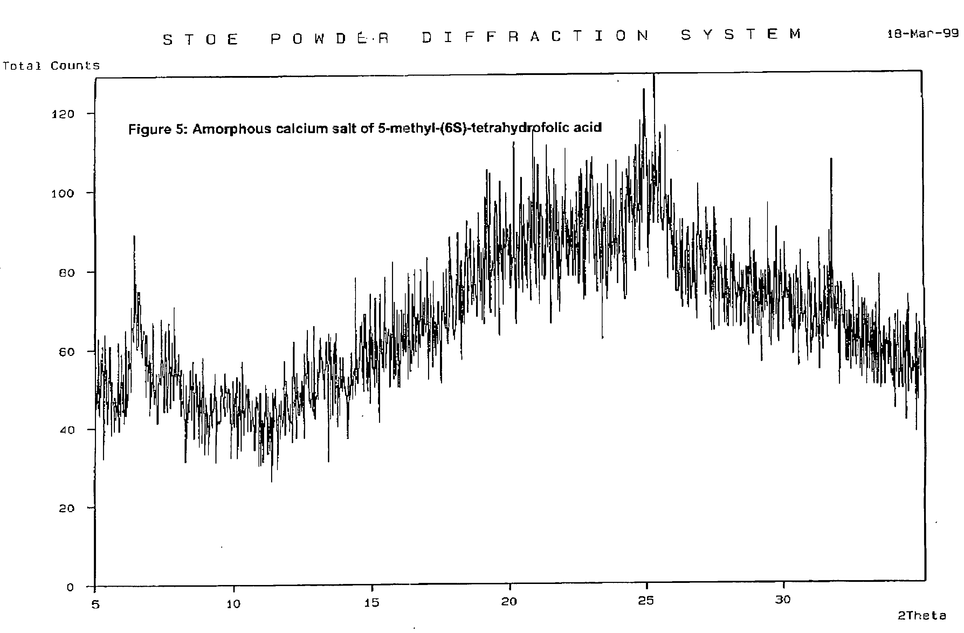

Beispiele von Spektren sind in Figure 1 (Typ I), Figure 2 (Typ II), Figure 3 (Typ III) und Figure 4 (Typ IV), bzw. in Table 1 (Typ I), Table 2 (Typ II), Table 3 (Typ III) und Table 4 (Typ IV) ersichtlich. Zum Vergleich wurde von einer amorphen Probe unter analogen Bedingungen ebenfalls ein Spektrum aufgenommen und als Figure 5 (amorph) aufgeführt.

Im Folgenden sind ausgewählte 2 Theta-Werte für die verschiedenen Kristallmodifikationen des kristallinen 5-Methyl-(6S)-tetrahydrofolsäure-Calcium-Salzes aufgelistet:

| **Typ** | **ausgewählte 2 Theta-Warte** |
|---|---|
| Typ I | 6.5, 13.3, 16.8 und 20.1 |
| Typ II | 5.3, 6.9, 18.7 und 21.1 |
| Typ III | 6.8, 10.2, 15.4 und 22.5 |
| Typ IV | 6.6, 15.9, 20.2 und 22.5 |

### Beispiel 3 [Löslichkeiten]

Die Löslichkeit von kristallinem 5-Methyl-(6S)-tetrahydrofolsäure-Calcium-Salz ist in der folgenden Tabelle aufgezeichnet:

| **Typ** | **Löslichkeit bei 20°C in** | |
|---|---|---|
| | **0.9% NaCl** | **Wasser** |
| Typ I | 1.6% | 1.1% |
| Typ II | 5.8% | 3.8% |
| Typ III | 1.5% | 1.0% |

### Beispiel 4 [amorphes 5-Methyl-(6S)-tetrahydrofolsäure-Calcium-Salz]

7.5 g 5-Methyl-(6S)-tetrahydrofolsäure werden unter Einleitung von N₂ bei Raumtemperatur in 75 ml Wasser eingetragen und mit Natronlauge 30% auf pH 12 eingestellt. Die so erhaltene klare Lösung wird mit Salzsäure 37% auf pH 7.5 eingestellt und mit einer Lösung von 7.15 g Calciumchlorid 6H₂O in 11.7 ml Wasser versetzt. Die entstandene weisse Suspension wird nach 5 Stunden ausrühren bei Raumtemperatur genutscht, mit Wasser überwaschen und bei 45°C im Vakuum getrocknet.

Man erhält 5.8 g weisses amorphes 5-Methyl-(6S)-tetrahydrofolsäure-Calcium-Salz mit einem Gehalt von 98.0% und einem 6S-Anteil von 99.6%.

Auch nach der Behandlung dieser Substanz bei 60°C in der Feuchtekammer können weder im Polarisationsmikroskop noch in einer Röntgendiffraktionsmessung kristalline Anteile bestimmt werden.

### Beispiel 5 [kristallines 5-Methyl-(6R,S)-tetrahydrofolsäure-Calcium-Salz]

70 g 5-Methyl-(6R,S)-tetrahydrofolsäure werden in 780 ml Wasser vorgelegt und mit 45.2 g NaOH 30% auf pH 7.5 eingestellt. Die klare leicht rötliche Lösung wird mit einer Lösung von 62.7 g Calciumchlorid·6H₂O in 140 ml Wasser versetzt, abfiltriert und mit wenig Wasser überwaschen. Das so erhaltene Rohprodukt wird in Wasser supspendiert und bei 90°C in einer Feuchtekammer während 24 Stunden behandelt.

Man erhält 74.0 g weisses kristallines 5-Methyl-(6R,S)-tetrahydrofolsäure-Calcium-Salz mit einem Gehalt von 99.1%.

### Beispiel 6 [kristallines 5-Methyl-(6R)-tetrahydrofolsäure-Calcium-Salz]

16.5 g 5-Methyl-(6R)-tetrahydrofolsäure werden in 100 ml Wasser von 92°C und 50 g Calciumchlorid·6H₂O vorgelegt. Die klare leicht gelbliche Suspension wird 10 Minuten bei 91 °C gerührt, abfiltriert, mit wenig Wasser überwaschen und bei 35°C im Vakuum getrocknet.

Man erhält 15.4 g hellbeiges kristallines 5-Methyl-(6R)-tetrahydrofolsäure-Calcium-Salz mit einem Gehalt von 97.9% und einem Wassergehalt von 7.8%.

### Beispiel 7 [Typ I]

130 kg Wasser werden vorgelegt und 12.8 kg 5-Methyl-(6S)-tetrahydrofolsäure eingetragen. Mit ca. 9.1 kg NaOH 30% wird der pH-Wert auf 11.6 und dann mit ca. 1.9 kg Salzsäure 37% auf 7.6 eingestellt. Zur klaren Lösung wird eine Suspension enthaltend 0.3 kg Kohle und 0.3 kg Cellflock zugegeben, filtriert und mit 13 l Wasser überwaschen. Das Filtrat wird mit einer Lösung enthaltend 8.3 kg Calciumchlorid·2H₂O versetzt, auf 90°C erhitzt und 30 Minuten gerührt. Das Produkt wird heiss abfiltriert und mit 2 x 20 kg Wasser überwaschen. Das so erhaltene feuchte Rohprodukt wird in 115 I Wasser aufgeschlämmt, auf 90°C erhitzt, sofort heiss abfiltriert, mit 2 x 20 kg Wasser überwaschen und bei 40°C im Vakuum getrocknet.

Man erhält 11.6 kg weisses kristallines 5-Methyl-(6S)-tetrahydrofolsäure-Calcium-Salz (Typ I) mit einer Reinheit von 99.0% und einem Wassergehalt von 14.5%.

### Belspiel 8 [Typ 1]

1600 ml Wasser werden vorgelegt und 194 g 5-Methyl-(6S)-tetrahydrofolsäure eingetragen. Mit ca. 80 ml NaOH 30% wird der pH-Wert auf 7.0 eingestellt. Zur klaren Lösung wird eine Suspension enthaltend 20 g Kohle und 20 g Cellflock in 190 ml Wasser zugegeben, filtriert und mit Wasser überwaschen. Das Filtrat wird mit einer 950 ml Calciumchlorid-Lösung 5.5 M versetzt, auf 90°C erhitzt und 60 Minuten gerührt. Das Produkt wird heiss abfiltriert und mit Wasser überwaschen und bei 45°C im Vakuum getrocknet.

Man erhält 156.2 g weisses kristallines 5-Methyl-(6S)-tetrahydrofolsäure-Calcium-Salz (Typ I) mit einer Reinheit von 99.7% und einem 6S-Anteil von 99.9%.

### Beispiel 9 [Typ I und Umwandlung in Typ II]

554 g Wasser werden vorgelegt und 53.1 g 5-Methyl-(6S)-tetrahydrofolsäure eingetragen. Mit NaOH 30% wird der pH-Wert auf 7.5 eingestellt. Zu klaren Lösung werden 1.3 g Kohle, 1.3 g Cellflock und 19,5 g Wasser zugegeben. Die Suspension wird filtriert und mit 55 ml Wasser überwaschen. Das Filtrat wird mit einer Lösung von 52.0 g Calciumchlorid·6H₂O in 84.6 g Wasser versetzt, auf 90°C erhitzt und mit 100 mg kristallinem 5-Methyltetrahydrofolsäure-Calcium-Salz angeimpft. Nach erfolgter Kristallisation wird das Produkt bei 90°C heiss abfiltriert und mit 2 x 103 g Wasser überwaschen. Das so erhaltene feuchte Rohprodukt wird in 480 ml Wasser aufgeschlämmt, auf 90°C erhitzt, sofort heiss abfiltriert, analog oben überwaschen und bei 45°C im Vakuum getrocknet.

Man erhält 47.5 g weisses kristallines 5-Methyl-(6S)-tetrahydrofolsäure-Calcium-Salz (Typ I) mit einer Reinheit von 98.8% und einem Wassergehalt von 12.2%.

Durch Trocknen bei 70°C im Vakuum während 30 Minuten kann diese Modifikation Typ I in die Modifikation Typ II mit einem Wassergehalt von 5.0% umgewandelt werden.

### Beispiel 10 [Typ III]

15.8 g 5-Methyl-(6S)-tetrahydrofolsäure-Calcium-Salz werden in 140 ml Wasser unter Einleitung von N₂ auf 95°C erhitzt, nach 30 Minuten bei 95°C wird die weisse Suspension heiss genutscht, mit Wasser überwaschen und bei 35°C im Vakuum getrocknet.

Man erhält 14.0 g weisses kristallines 5-Methyl-(6S)-tetrahydrofolsäure-Calcium-Salz (Typ III) mit einem Gehalt von 98.9% und einem 6S-Anteil von 99.9%.

### Beispiel 11 [Typ IV]

20.0 g 5-Methyl-(6S)-tetrahydrofolsäure-Calcium-Salz werden in 180 ml Wasser unter Einleitung von N₂ auf 100°C erhitzt, nach 30 Minuten bei 100°C wird die weisse Suspension heiss genutscht, mit Wasser überwaschen und bei 25°C im Vakuum getrocknet.

Man erhält 16.9 g weisses kristallines 5-Methyl-(6S)-tetrahydrofolsäure-Calcium-Salz (Typ IV) mit einem Gehalt von 98.3% und einem Wassergehalt von 9.9%.

Durch Trocknen bei 65°C im Vakuum kann der Wassergehalt dieses Produktes auf 5.5% reduziert werden, ohne dass dabei eine andere Kristallmodifikation erhalten wird.

## Patentansprüche

1. Kristalline Salze der 5-Methyl-(6R,S)-, -(6S)- und -(6R)-tetrahydrofolsäure.

2. Knstalline Salze der 5-Methyl-(6S)- und (6R)-tetrahydrofolsäure.

3. Kristallines Calcium-Salz der 5-Methyl-(6S)- und -(6R)-tetrahydrofolsäure.

4. Kristallines Calcium-Salz der 5-Methyl-(6S)-tetrahydrofolsäure mit 2 Theta-Werten von 6.5, 13.3, 16.8 und 20.1.

5. Kristallines Calcium-Salz der 5-Methyl-(6S)-tetrahydrofolsäure mit 2 Theta-Werten von 5.3, 6.9, 18.7 und 21.1.

6. Kristallines Calcium-Salz der 5-Methyl-(6S)-tetrahydrofolsäure mit 2 Theta-Werten von 6.8, 10.2, 15.4 und 22.5.

7. Kristallines Calcium-Salz der 5-Methyl-(6S)-tetrahydrofolsäure mit 2 Theta-Werten von 6.6, 15.9, 20.2 und 22.5.

8. Verfahren zur Herstellung von kristallinen Salzen der 5-Methyl-(6R,S)-, -(6S)- und 5-Methyl-(6R)-tetrahydrofolsäure, **dadurch gekennzeichnet, dass** man 5-Methyl-(6R,S)-, -(6S)- oder -(6R)-tetrahydrofolsäure-Salze aus einem polaren Medium nach einer Temperaturbehandlung kristallisiert.

9. Verfahren nach Anspruch 8, **dadurch gekennzeichnet, dass** die Kristallisation nach einer Temperaturbehandlung von über 60°C erfolgt.

10. Verfahren nach Anspruch 8, **dadurch gekennzeichnet, dass** die Kristallisation nach einer Temperaturbehandlung von über 85°C erfolgt.

11. Verfahren nach Anspruch 8, **dadurch gekennzeichnet, dass** die Kristallisation aus einer Lösung durchgeführt wird.

12. Verfahren nach Anspruch 8, **dadurch gekennzeichnet, dass** die Kristallisation aus einer Suspension durchgeführt wird.

13. Verfahren nach Anspruch 11 oder 12, **dadurch gekennzeichnet, dass** die Kristallisation aus Wasser oder einem Gemisch aus Wasser und einem mit Wasser mischbaren organischen Lösungsmittel durchgeführt wird.

14. Verwendung von kristallinen Salzen der 5-Methyl-(6S)- oder -(6R)-tetrahydrofolsäure als Bestandteil zur Herstellung von Arzneimitteln oder als Nahrungsmittelergänzungsstoff.

15. Zubereitungen enthaltend kristalline Salze der 5-Methyl-(6S)- oder -(6R)-tetrahydrofolsäure.
